# EUROPÄISCHE PATENTSCHRIFT

(11) **EP 1 320 412 B1**
(45) Veröffentlichungstag und Bekanntmachung des Hinweises auf die Patenterteilung: **04.01.2006**
(21) Anmeldenummer: 01980403.8
(22) Anmeldetag: 18.09.2001
(51) Int. Cl.: B01J 13/04

(54) **MIKROKAPSELPULVER**
MICROCAPSULE POWDER
POUDRE DE MICROCAPSULES

(30) Priorität: 25.09.2000 FR 0012168
(43) Veröffentlichungstag der Anmeldung: 25.06.2003
(73) Patentinhaber: Cognis France, S.A.S., 31360 Boussens (FR)
(72) Erfinder: GRISONI, Philippe, F-54760 Bey sur Seille (FR)
(74) Vertreter: Fabry, Bernd
(86) Internationale Anmeldenummer: PCT/EP2001/010765
(87) Internationale Veröffentlichungsnummer: WO 2002/024319

(56) Entgegenhaltungen:
- EP-A- 1 064 910
- DE-C- 4 122 591
- FR-A- 2 775 441

## Beschreibung

### Gebiet der Erfindung

Die Erfindung befindet sich auf dem Gebiet der Verkapselung von Wirkstoffen und betrifft hydrophobierte Pulver bestehend aus Mikro- und/oder Nanokapseln, sowie ein Verfahren zu deren Herstellung und ihre Verwendung in kosmetischen und pharmazeutischen Zubereitungen.

### Stand der Technik

Unter dem Begriff "Mikrokapsel" werden sphärische Aggregate mit einem Durchmesser im Bereich von etwa 1 bis etwa 5000 µm und unter "Nanokapseln" unterhalb 1 µm verstanden, die mindestens einen festen oder flüssigen Kern enthalten, der von mindestens einer kontinuierlichen Hülle umschlossen ist. Genauer gesagt handelt es sich um mit filmbildenden Polymeren umhüllte feindisperse flüssige oder feste Phasen, bei deren Herstellung sich die Polymere nach Emulgierung und Koazervation oder Grenzflächenpolymerisation auf dem einzuhüllenden Material niederschlagen. Nach einem anderen Verfahren werden flüssige Wirkstoffe in einer Matrix aufgenommen ("microsponge"), die als Mikropartikel zusätzlich mit filmbildenden Polymeren umhüllt sein können. Übergänge zwischen Mikro- bzw. Nanokapseln, in denen Stoffe durch eine Membran umhüllt sind (Reservoir System) und Mikro- bzw. Nanopartikel, in denen die Wirkstoffe in der Trägermatrix dispergiert oder gelöst vorliegen (Matrix System) ergeben sich durch das jeweilige Herstellungsverfahren. Neben einkernigen Mikrokapseln sind auch mehrkernige Aggregate, auch Mikrosphären genannt, bekannt, die zwei oder mehr Kerne im kontinuierlichen Hüllmaterial verteilt enthalten. Ein- oder mehrkernige Mikrokapseln können zudem von einer zusätzlichen zweiten, dritten etc. Hülle umschlossen sein. Die Hülle kann aus natürlichen, halbsynthetischen oder synthetischen Materialien bestehen. Natürlich Hüllmaterialien sind beispielsweise Gummi Arabicum, Agar-Agar, Agarose, Maltodextrine, Alginsäure bzw. ihre Salze, z.B. Natrium- oder Calciumalginat, Fette und Fettsäuren, Cetylalkohol, Collagen, Chitosan, Lecithine, Gelatine, Albumin, Schellack, Polysaccaride, wie Stärke oder Dextran, Polypeptide, Proteinghydrolysate, Sucrose und Wachse. Halbsynthetische Hüllmaterialien sind unter anderem chemisch modifizierte Cellulosen, insbesondere Celluloseester und -ether, z.B. Celluloseacetat, Ethylcellulose, Hydroxypropylcellulose, Hydroxypropylmethylcellulose und Carboxymethylcellulose, sowie Stärkederivate, insbesondere Stärkeether und -ester. Synthetische Hüllmaterialien sind beispielsweise Polymere wie Polyacrylate, Polyamide, Polyvinylalkohol oder Polyvinylpyrrolidon.

Beispiele für Mikrokapseln des Stands der Technik sind folgende Handelsprodukte (in Klammern angegeben ist jeweils das Hüllmaterial): *Hallcrest Microcapsules* (Gelatine, Gummi Arabicum), *Coletica* Thalaspheres (maritimes Collagen), *Lipotec Millicapseln* (Alginsäure, Agar-Agar), *Induchem Unispheres* (Lactose, mikrokristalline Cellulose, Hydroxypropylmethylcellulose); *Unicerin* C30 (Lactose, mikrokristalline Cellulose, Hydroxypropylmethylcellulose), *Kobo Glycospheres* (modifizierte Stärke, Fettsäureester, Phospholipide), *Softspheres* (modifiziertes Agar-Agar) und *Kuhs Probiol Nanospheres* (Phospholipide).

In diesem Zusammenhang sei auch auf die deutsche Patentanmeldung **DE 19712978 A1** (Henkel) hingewiesen, aus der Chitosanmikrosphären bekannt sind, die man erhält, indem man Chitosane oder Chitosanderivate mit Ölkörpern vermischt und diese Mischungen in alkalisch eingestellte Tensidlösungen einbringt. Aus der deutschen Patentanmeldung **DE 19756452 A1** (Henkel) ist ferner auch die Verwendung von Chitosan als Verkapselungsmaterial für Tocopherol bekannt. Gegenstand der Anmeldung **EP 99 122 906** sind Mikrokapseln mit mittleren Durchmessern im Bereich von 0,1 bis 500, vorzugsweise 25 bis 250 und insbesondere 50 bis 100 µm, bestehend aus einer Hüllmembran aus Stärke und Chitosanen.

Die auf unterschiedliche Weise hergestellten Mikro- und Nanokapseln können dann durch Trocknung über Lyophilisation, Wirbelbetttrocknung oder durch Entwässerung zu einem pulverförmigen Endprodukt umgearbeitet werden (**FR 2775441 B1** und **EP 99 122 906**). Vielfach beinhaltet diese Weiterverarbeitung jedoch umständliche, kosten- und zeitaufwendige Prozesse und führt nicht selten zu einer vorzeitigen Zerstörung der Mikro- und Nanokapseln. Da die Mikrokapseln hydrophile Oberflächeneigenschaften haben, lassen sie sich häufig schlecht in lipophile Zubereitungen einarbeiten.

Die Anwendung sogenannter Dispersionshilfsstoffe (kolloidales Silicium), die zu einer Optimierung der Fließfähigkeit der Pulver beiträgt, wird erst nach der Gewinnung bereits isolierter Pulver angewendet. Ein Vorteil ähnlicher Formulierungen, die Anwendung in Kosmetika finden, ist die Regulierung von fettabsorbierenden gegenüber austrocknenden Eigenschaften **(US 5948417).** In der Patentschrift **US 5356617** wird beschrieben wie die unzureichende Verarbeitung hydrophiler Pigmente durch Herstellung von Mikropartikeln aus organischem Polymer, anorganischen Pigmenten und einem Binder verbessert werden kann. So können auch ölige Substanzen und lipophile Träger zur besseren Verarbeitung in Pulver überführt werden, die dann einfach in kosmetische Mittel eingearbeitet werden (**EP 0659403** und **US 4164563**). Der Einsatz von Mikro-und/oder Nanokapseln ergibt jedoch größere Verarbeitungsprobleme.

Die Freisetzung der Wirkstoffe aus den Mikrokapseln erfolgt üblicherweise während der Anwendung der sie enthaltenden Zubereitungen durch Zerstörung der Hülle infolge mechanischer, thermischer, chemischer oder enzymatischer Einwirkung oder durch Diffusion. Von Nachteil ist dabei, dass die Mikrokapseln die kontrollierte Freisetzung der Wirkstoffe aus ihrem Innern nicht oder nur in unzureichendem Maße zulassen und die Kapseln eine ungenügende Stabilität in Gegenwart von Tensiden, zumal anionischen Tensiden, Salzen oder durch mechanische Belastung aufweisen.

Aufgabe der vorliegenden Erfindung ist es daher, eine stabile pulverförmige Formulierung bestehend aus hydrophil verkapselten Wirk- und Hilfsstoffen zur Verfügung zu stellen, die sich leicht in wasserfreie Formulierungen einarbeiten lässt. Dabei soll die Herstellung durch einfache ökonomische Verfahren vollzogen werden und zusätzlich eine Steuerung des Freisetzungsverhaltens ermöglichen, sowie eine lange Lagerbeständigkeit der Formulierung gewährleisten.

### Beschreibung der Erfindung

Gegenstand der Erfindung sind hydrophobierte Pulver bestehend aus Mikro- und/oder Nanokapseln, dadurch erhältlich, dass man
(a) eine wäßrige Lösung mindestens eines Polymers in Gegenwart eines W/O-Emulgators in einem Öl bei einer Temperatur oberhalb des Gelpunktes der Polymerlösung dispergiert,
(b) die Dispersion unter Rühren auf eine Temperatur unterhalb des Gelpunktes abkühlt,
(c) die dabei entstandenen Mikro- und/oder Nanokapseln durch Dekantieren isoliert, und
(d) die so erhaltene ölige Dispersion mit einem ölabsorbierenden Hilfsstoff versetzt.

Überraschenderweise wurde gefunden, dass hydrophobierte Pulver durch einfaches Mischen einer öligen Dispersion aus Mikro- und/oder Nanokapseln mit ölabsorbierenden Hilfsstoffen herzustellen sind, und dass diese Formulierung eine stabile Pulverform mit einem hohen Gehalt an wasserlöslichen Aktivstoffen darstellt. Durch die Schicht der anhaftenden ölabsorbierenden Hilfsstoffe werden die hydrophilen Mikro-und/oder Nanokapseln hydrophobiert, vor frühzeitiger Hydratation und Oxidation geschützt. Die Formulierung kann einfach in wasserfreie Zubereitungen eingearbeitet werden und zeichnet sich durch eine gute Lagerbeständigkeit ohne Verkleben der hydrophilen Mikro- bzw. Nanokapseln aus. Weiterhin kann durch Variation von Art und Menge des ölabsorbierenden Hilfsstoffes die Freigabe der eingekapselten Aktivstoffe gesteuert werden.

Ein weiterer Gegenstand der Erfindung betrifft das Verfahren zur Herstellung von hydrophobierten Pulvern bestehend aus Mikro- und/oder Nanokapseln , bei dem man
(a) eine erwärmte wäßrige Lösung mindestens eines Polymers in Gegenwart eines W/O-Emulgators in einem Öl bei einer Temperatur oberhalb des Gelpunktes der Polymerlösung dispergiert,
(b) die Dispersion unter Rühren auf eine Temperatur unterhalb des Gelpunktes abkühlt,
(c) die dabei entstandenen Mikro- und/oder Nanokapseln durch Dekantieren isoliert, und
(d) die so erhaltene ölige Dispersion mit einem ölabsorbierenden Hilfsstoff versetzt,

sowie die Verwendung der hydrophobierten Pulver in kosmetischen und/oder pharmazeutischen Zubereitungen und Wasch- und Reinigungsmitteln.

### Mikro- und/oder Nanokapseln

Die in den erfindungsgemäßen hydrophobierten Pulvern vorliegenden Mikro- und/oder Nanokapseln entstehen bei der Abkühlung einer Polymerhaltigen W/O-Emulsion. Die nach der Abkühlung vorliegende ölige Dispersion enthält nach dem Abdekantieren einen Feststoffanteil von 85 bis 95 Gew. % bestehend aus hydrophilen Mikro-und/oder Nanokapseln und 5 bis 15 Gew. % des eingesetzten Öles in der äußeren Phase.
Die Mikro- und/oder Nanokapseln weisen darin einen mittleren Durchmesser im Bereich von 0,1 bis 500 µm, vorzugsweise 25 bis 100 und insbesondere 10 bis 50 µm auf. Besonders Partikel unter 50 µm Teilchendurchmesser ähneln der Partikelgröße vieler Pulverformulierungen in der dekorativen Kosmetik und können daher unauffällig untergemischt werden.

### Polymere

Als Polymere zur Bildung der Kapselmatrix werden vorwiegend hydrophile Materialien eingesetzt.
Die Hülle kann aus natürlichen, halbsynthetischen oder synthetischen Materialien bestehen.
Natürlich Hüllmaterialien sind beispielsweise Gummi Arabicum, Agar-Agar, Agarose, Carrageen, Maltodextrine, Alginsäure bzw. ihre Salze, z.B. Natrium- oder Calciumalginat, Fette und Fettsäuren, Cetylalkohol, Collagen, Chitosan, Lecithine, Gelatine, Gluten, Albumin, Schellack, Polysaccaride, wie Stärke oder Dextran, Polypeptide, Proteinhydrolysate, Sucrose, Xanthan oder Gellan Gum.
Halbsynthetische Hüllmaterialien sind unter anderem chemisch modifizierte Cellulosen, insbesondere Celluloseester und -ether, z.B. Celluloseacetat, Ethylcellulose, Hydroxypropylcellulose, Hydroxypropylmethylcellulose und -phthalat, Methylcellulose und Carboxymethylcellulose, sowie Stärkederivate, insbesondere Stärkeether und -ester und Stearate.
Zu den synthetischen Hüllmaterialien sind beispielsweise Polymere wie Polyacrylate, Polyamide, Polyvinylalkohol, Polyglycolate, Polyoxyethylene, Polylactate, Polyglutamate, Polyimide oder Polyvinylpyrrolidon zu zählen.

### Ölkörper

Als Ölkörper, die als äußere Phase bei der Mikro- und/oder Nanokapselherstellung eingesetzt werden können, kommen pflanzliche, tierische, halbsynthetische und synthetische Öle in Betracht, beispielsweise Guerbetalkohole auf Basis von Fettalkoholen mit 6 bis 18, vorzugsweise 8 bis 10 Kohlenstoffatomen, Ester von linearen C₆-C₂₂-Fettsäuren mit linearen C₆-C₂₂-Fettalkoholen, Ester von verzweigten C₆-C₁₃-Carbonsäuren mit linearen C₆-C₂₂-Fettalkoholen, wie z.B. Myristylmyristat, Myristylpalmitat, Myristylstearat, Myristylisostearat, Myristyloleat, Myristylbehenat, Myristylerucat, Cetylmyristat, Cetylpalmitat, Cetylstearat, Cetylisostearat, Cetyloleat, Cetylbehenat, Cetylerucat, Stearylmyristat, Stearylpalmitat, Stearylstearat, Stearylisostearat, Stearyloleat, Stearylbehenat, Stearylerucat, Isostearylmyristat, Isostearylpalmitat, Isostearylstearat, Isostearylisostearat, Isostearyloleat, Isostearylbehenat, Isostearyloleat, Oleylmyristat, Oleylpalmitat, Oleylstearat, Oleyl-isostearat, Oleyloleat, Oleylbehenat, Oleylerucat, Behenylmyristat, Behenylpalmitat, Behenylstearat, Behenylisostearat, Behenyloleat, Behenylbehenat, Behenylerucat, Erucylmyristat, Erucylpalmitat, Erucylstearat, Erucylisostearat, Erucyloleat, Erucylbehenat und Erucylerucat. Daneben eignen sich Ester von linearen C₆-C₂₂-Fettsäuren mit verzweigten Alkoholen, insbesondere 2-Ethylhexanol, Ester von Hydroxycarbonsäuren mit linearen oder verzweigten C₆-C₂₂-Fettalkoholen, insbesondere Dioctyl Malate, Ester von linearen und/oder verzweigten Fettsäuren mit mehrwertigen Alkoholen (wie z.B. Propylenglycol, Dimerdiol oder Trimertriol) und/oder Guerbetalkoholen, Triglyceride auf Basis C₆-C₁₀-Fettsäuren, flüssige Mono-/Di-/Triglyceridmischungen auf Basis von C₆-C₁₈-Fettsäuren, Ester von C₆-C₂₂-Fettalkoholen und/oder Guerbetalkoholen mit aromatischen Carbonsäuren, insbesondere Benzoesäure, Ester von C₂-C₁₂-Dicarbonsäuren mit linearen oder verzweigten Alkoholen mit 1 bis 22 Kohlenstoffatomen oder Polyolen mit 2 bis 10 Kohlenstoffatomen und 2 bis 6 Hydroxylgruppen, pflanzliche Öle, verzweigte primäre Alkohole, substituierte Cyclohexane, lineare und verzweigte C₆-C₂₂-Fettalkoholcarbonate, Guerbetcarbonate, Ester der Benzoesäure mit linearen und/oder verzweigten C₆-C₂₂-Alkoholen (z.B. Finsolv® TN), lineare oder verzweigte, symmetrische oder unsymmetrische Dialkylether mit 6 bis 22 Kohlenstoffatomen pro Alkylgruppe, Ringöffnungsprodukte von epoxidierten Fettsäureestern mit Polyolen, Siliconöle und/oder aliphatische bzw. naphthenische Kohlenwasserstoffe, wie z.B. wie Squalan, Squalen oder Dialkylcyclohexane.

### Ölabsorber

Der ölabsorbierende Hilfsstoff d) wird in Mengen von 5 bis 35 Gew. %, vorzugsweise 10 bis 30 Gew. % besonders bevorzugt 15 bis 25 Gew. % bezogen auf die Menge des hydrophobierten Pulvers eingesetzt. Alle kosmetisch und pharmazeutisch verträglichen Substanzen können dazu eingesetzt werden, wie zum Beispiel organische und anorganische Pigmente, Dimethicone, Dimethicon Cross Polymere, Stärke, Methacrylatverbindungen und Acrylatcopolymere, Polymethylmethacrylate, Silicate, Magnesiumstearat, Zinkstearat, Magnesiumcarbonat können als Ölabsorber.

### Wirkstoffe für kosmetische und pharmazeutische Anwendungen

Typische Beispiele für Wirkstoffe, wie sie im Bereich der kosmetischen und pharmazeutischen Zubereitungen eingesetzt werden sind Pflanzenextrakte, Wirkstoffe mit antibakteriellen, Anti-Akne- und keratolytischen Eigenschaften, Tenside, kosmetische Öle, Perlglanzwachse, Stabilisatoren, biogene Wirkstoffe, Vitamine, Deodorantien, Antitranspirantien, Antischuppenmittel, UV-Lichtschutzfaktoren, Antioxidantien, Konservierungsmittel, Insektenrepellentien, Selbstbräuner, Typrosininhibitoren (Depigmentierungsmittel), Parfümöle und Farbstoffe.

Als oberflächenaktive Stoffe können anionische, nichtionische, kationische und/oder amphotere bzw. amphotere Tenside verkapselt werden. Typische Beispiele für anionische Tenside sind Seifen, Alkylbenzolsulfonate, Alkansulfonate, Olefinsulfonate, Alkylethersulfonate, Glycerinethersulfonate, α-Methylestersulfonate, Sulfofettsäuren, Alkylsulfate, Fettalkoholethersulfate, Glycerinethersulfate, Fettsäureethersulfate, Hydroxymischethersulfate, Monoglycerid(ether)sulfate, Fettsäureamid(ether)sulfate, Mono- und Dialkylsulfosuccinate, Mono- und Dialkylsulfosuccinamate, Sulfotriglyceride, Amidseifen, Ethercarbonsäuren und deren Salze, Fettsäureisethionate, Fettsäuresarcosinate, Fettsäuretauride, N-Acylaminosäuren, wie beispielsweise Acyllactylate, Acyltartrate, Acylglutamate und Acylaspartate, Alkyloligoglucosidsulfate, Proteinfettsäurekondensate (insbesondere pflanzliche Produkte auf Weizenbasis) und Alkyl(ether)phosphate. Sofern die anionischen Tenside Polyglycoletherketten enthalten, können diese eine konventionelle, vorzugsweise jedoch eine eingeengte Homologenverteilung aufweisen. Typische Beispiele für nichtionische Tenside sind Fettalkoholpolyglycolether, Alkylphenolpolyglycolether, Fettsäurepolyglycolester, Fettsäureamidpolyglycolether, Fettaminpolyglycolether, alkoxylierte Triglyceride, Mischether bzw. Mischformale, gegebenenfalls partiell oxidierte Alk(en)yloligoglykoside bzw. Glucoronsäurederivate, Fettsäure-N-alkylglucamide, Proteinhydrolysate (insbesondere pflanzliche Produkte auf Weizenbasis), Polyolfettsäureester, Zuckerester, Sorbitanester, Polysorbate und Aminoxide. Sofern die nichtionischen Tenside Polyglycoletherketten enthalten, können diese eine konventionelle, vorzugsweise jedoch eine eingeengte Homologenverteilung aufweisen. Typische Beispiele für kationische Tenside sind quartäre Ammoniumverbindungen, wie beispielsweise das Dimethyldistearylammoniumchlorid, und Esterquats, insbesondere quaternierte Fettsäuretrialkanolaminestersalze. Typische Beispiele für amphotere bzw. zwitterionische Tenside sind Alkylbetaine, Alkylamidobetaine, Aminopropionate, Aminoglycinate, Imidazoliniumbetaine und Sulfobetaine. Bei den genannten Tensiden handelt es sich ausschließlich um bekannte Verbindungen. Hinsichtlich Struktur und Herstellung dieser Stoffe sei auf einschlägige Übersichtsarbeiten beispielsweise J.Falbe (ed.), "Surfactants in Consumer Products", Springer Verlag, Berlin, 1987, S. 54-124 oder J.Falbe (ed.), "Katalysatoren, Tenside und Mineralöladditive", Thieme Verlag, Stuttgart, 1978, S. 123-217 verwiesen.

Als **Periglanzwachse** kommen beispielsweise in Frage: Alkylenglycolester, speziell Ethylenglycoldistearat; Fettsäurealkanolamide, speziell Kokosfettsäurediethanolamid; Partialglyceride, speziell Stearinsäuremonoglycerid; Ester von mehrwertigen, gegebenenfalls hydroxysubstituierte Carbonsäuren mit Fettalkoholen mit 6 bis 22 Kohlenstoffatomen, speziell langkettige Ester der Weinsäure; Fettstoffe, wie beispielsweise Fettalkohole, Fettketone, Fettaldehyde, Fettether und Fettcarbonate, die in Summe mindestens 24 Kohlenstoffatome aufweisen, speziell Lauron und Distearylether; Fettsäuren wie Stearinsäure, Hydroxystearinsäure oder Behensäure, Ringöffnungsprodukte von Olefinepoxiden mit 12 bis 22 Kohlenstoffatomen mit Fettalkoholen mit 12 bis 22 Kohlenstoffatomen und/oder Polyolen mit 2 bis 15 Kohlenstoffatomen und 2 bis 10 Hydroxylgruppen sowie deren Mischungen.

Als **Stabilisatoren** können Metallsalze von Fettsäuren, wie z.B. Magnesium-, Aluminium- und/oder Zinkstearat bzw. -ricinoleat eingesetzt werden.

Unter **biogenen Wirkstoffen** sind beispielsweise Tocopherol, Tocopherolacetat, Tocopherolpalmitat, Ascorbinsäure, Desoxyribonucleinsäure, Retinol, Bisabolol, Allantoin, Phytantriol, Panthenol, AHA-Säuren, Kojisäure, Aminosäuren, Ceramide, Pseudoceramide, essentielle Öle, Pflanzenextrakte und Vitaminkomplexe zu verstehen.

Kosmetische **Deodorantien** (Desodorantien) wirken Körpergerüchen entgegen, überdecken oder beseitigen sie. Körpergerüche entstehen durch die Einwirkung von Hautbakterien auf apokrinen Schweiß, wobei unangenehm riechende Abbauprodukte gebildet werden. Dementsprechend enthalten Deodorantien Wirkstoffe, die als keimhemmende Mittel, Enzyminhibitoren, Geruchsabsorber oder Geruchsüberdecker fungieren.

Als **keimhemmende Mittel** sind grundsätzlich alle gegen grampositive Bakterien wirksamen Stoffe geeignet, wie z. B. 4-Hydroxybenzoesäure und ihre Salze und Ester, N-(4-Chlorphenyl)-N'-(3,4 dichlorphenyl)harnstoff, 2,4,4'-Trichlor-2'-hydroxydiphenylether (Triclosan), 4-Chlor-3,5-dimethylphenol, 2,2'-Methylen-bis(6-brom-4-chlorphenol), 3-Methyl-4-(1-methylethyl)phenol, 2-Benzyl-4-chlorphenol, 3-(4-Chlorphenoxy)-1,2-propandiol, 3-lod-2-propinylbutylcarbamat, Chlorhexidin, 3,4,4'-Trichlorcarbanilid (TTC), antibakterielle Riechstoffe, Thymol, Thymianöl, Eugenol, Nelkenöl, Menthol, Minzöl, Farnesol, Phenoxyethanol, Glycerinmonolaurat (GML), Diglycerinmonocaprinat (DMC), Salicylsäure-N-alkylamide wie z. B. Salicylsäure-n-octylamid oder Salicylsäure-n-decylamid.

Als **Enzyminhibitoren** sind beispielsweise Esteraseinhibitoren geeignet. Hierbei handelt es sich vorzugsweise um Trialkylcitrate wie Trimethylcitrat, Tripropylcitrat, Triisopropylcitrat, Tributylcitrat und insbesondere Triethylcitrat (Hydagen® CAT, Henkel KGaA, Düsseldorf/FRG). Die Stoffe inhibieren die Enzymaktivität und reduzieren dadurch die Geruchsbildung. Weitere Stoffe, die als Esteraseinhibitoren in Betracht kommen, sind Sterolsulfate oder -phosphate, wie beispielsweise Lanosterin-, Cholesterin-, Campesterin-, Stigmasterin- und Sitosterinsulfat bzw -phosphat, Dicarbonsäuren und deren Ester, wie beispielsweise Glutarsäure, Glutarsäuremonoethylester, Glutarsäurediethylester, Adipinsäure, Adipinsäuremonoethylester, Adipinsäurediethylester, Malonsäure und Malonsäurediethylester, Hydroxycarbnonsäuren und deren Ester wie beispielsweise Citronensäure, Äpfelsäure, Weinsäure oder Weinsäurediethylester, sowie Zinkglycinat.

Als **Geruchsabsorber** eignen sich Stoffe, die geruchsbildende Verbindungen aufnehmen und weitgehend festhalten können. Sie senken den Partialdruck der einzelnen Komponenten und verringern so auch ihre Ausbreitungsgeschwindigkeit. Wichtig ist, daß dabei Parfums unbeeinträchtigt bleiben müssen. Geruchsabsorber haben keine Wirksamkeit gegen Bakterien. Sie enthalten beispielsweise als Hauptbestandteil ein komplexes Zinksalz der Ricinolsäure oder spezielle, weitgehend geruchsneutrale Duftstoffe, die dem Fachmann als "Fixateure" bekannt sind, wie z. B. Extrakte von Labdanum bzw. Styrax oder bestimmte Abietinsäurederivate. Als Geruchsüberdecker fungieren Riechstoffe oder Parfümöle, die zusätzlich zu ihrer Funktion als Geruchsüberdecker den Deodorantien ihre jeweilige Duftnote verleihen. Als Parfümöle seien beispielsweise genannt Gemische aus natürlichen und synthetischen Riechstoffen. Natürliche Riechstoffe sind Extrakte von Blüten, Stengeln und Blättern, Früchten, Fruchtschalen, Wurzeln, Hölzern, Kräutern und Gräsern, Nadeln und Zweigen sowie Harzen und Balsamen. Weiterhin kommen tierische Rohstoffe in Frage, wie beispielsweise Zibet und Castoreum. Typische synthetische Riechstoffverbindungen sind Produkte vom Typ der Ester, Ether, Aldehyde, Ketone, Alkohole und Kohlenwasserstoffe. Riechstoffverbindungen vom Typ der Ester sind z.B. Benzylacetat, p-tert.-Butylcyclohexylacetat, Linalylacetat, Phenylethylacetat, Linalylbenzoat, Benzylformiat, Allylcyclohexylpropionat, Styrallylpropionat und Benzylsalicylat. Zu den Ethern zählen beispielsweise Benzylethylether, zu den Aldehyden z.B. die linearen Alkanale mit 8 bis 18 Kohlenstoffatomen, Citral, Citronellal, Citronellyloxyacetaldehyd, Cyclamenaldehyd, Hydroxycitronellal, Lilial und Bourgeonal, zu den Ketonen z.B. die Jonone und Methylcedrylketon, zu den Alkoholen Anethol, Citronellol, Eugenol, Isoeugenol, Geraniol, Linalool, Phenylethylalkohol und Terpineol, zu den Kohlenwasserstoffen gehören hauptsächlich die Terpene und Balsame. Bevorzugt werden jedoch Mischungen verschiedener Riechstoffe verwendet, die gemeinsam eine ansprechende Duftnote erzeugen. Auch ätherische Öle geringerer Flüchtigkeit, die meist als Aromakomponenten verwendet werden, eignen sich als Parfümöle, z.B. Salbeiöl, Kamillenöl, Nelkenöl, Melissenöl, Minzenöl, Zimtblätteröl, Lindenblütenöl, Wacholderbeerenöl, Vetiveröl, Olibanöl, Galbanumöl, Labdanumöl und Lavandinöl. Vorzugsweise werden Bergamotteöl, Dihydromyrcenol, Lilial, Lyral, Citronellol, Phenylethylalkohol, α-Hexylzimtaldehyd, Geraniol, Benzylaceton, Cyclamenaldehyd, Linalool, Boisambrene Forte, Ambroxan, Indol, Hedione, Sandelice, Citronenöl, Mandarinenöl, Orangenöl, Allylamylglycolat, Cyclovertal, Lavandinöl, Muskateller Salbeiöl, β-Damascone, Geraniumöl Bourbon, Cyclohexylsalicylat, Vertofix Coeur, Iso-E-Super, Fixolide NP, Evernyl, Iraldein gamma, Phenylessigsäure, Geranylacetat, Benzylacetat, Rosenoxid, Romilat, Irotyl und Floramat allein oder in Mischungen, eingesetzt.

**Antitranspirantien** (Antiperspirantien) reduzieren durch Beeinflussung der Aktivität der ekkrinen Schweißdrüsen die Schweißbildung, und wirken somit Achselnässe und Körpergeruch entgegen. Wässrige oder wasserfreie Formulierungen von Antitranspirantien enthalten typischerweise folgende Inhaltsstoffe:
➢ adstringierende Wirkstoffe,
➢ Ölkomponenten,
➢ nichtionische Emulgatoren,
➢ Coemulgatoren,
➢ Konsistenzgeber,
➢ Hilfsstoffe wie z. B. Verdicker oder Komplexierungsmittel und/oder
➢ nichtwässrige Lösungsmittel wie z. B. Ethanol, Propylenglykol und/oder Glycerin.

Als adstringierende Antitranspirant-Wirkstoffe eignen sich vor allem Salze des Aluminiums, Zirkoniums oder des Zinks. Solche geeigneten antihydrotisch wirksamen Wirkstoffe sind z.B. Aluminiumchlorid, Aluminiumchlorhydrat, Aluminiumdichlorhydrat, Aluminiumsesquichlorhydrat und deren Komplexverbindungen z. B. mit Propylenglycol-1,2. Aluminiumhydroxyallantoinat, Aluminiumchloridtartrat, Aluminium-Zirkonium-Trichlorohydrat, Aluminium-Zirkonium-tetrachlorohydrat, Aluminium-Zirkonium-pentachlorohydrat und deren Komplexverbindungen z. B. mit Aminosäuren wie Glycin. Daneben können in Antitranspirantien übliche öllösliche und wasserlösliche Hilfsmittel in geringeren Mengen enthalten sein. Solche öllöslichen Hilfsmittel können z.B. sein:
➢ entzündungshemmende, hautschützende oder wohlriechende ätherische Öle,
➢ synthetische hautschützende Wirkstoffe und/oder
➢ öllösliche Parfümöle.

Übliche wasserlösliche Zusätze sind z.B. Konservierungsmittel, wasserlösliche Duftstoffe, pH-Wert-Stellmittel, z.B. Puffergemische, wasserlösliche Verdickungsmittel, z.B. wasserlösliche natürliche oder synthetische Polymere wie z.B. Xanthan-Gum, Hydroxyethylcellulose, Polyvinylpyrrolidon oder hochmolekulare Polyethylenoxide.

Als **Antischuppenmittel** können Climbazol, Octopirox, Ketokonazol und Zinkpyrethion eingesetzt werden.

Unter **UV-Lichtschutzfaktoren** sind beispielsweise bei Raumtemperatur flüssig oder kristallin vorliegende organische Substanzen (Lichtschutzfilter) zu verstehen, die in der Lage sind, ultraviolette Strahlen zu absorbieren und die aufgenommene Energie in Form längerwelliger Strahlung, z.B. Wärme wieder abzugeben. UVB-Filter können öllöslich oder wasserlöslich sein. Als öllösliche Substanzen sind z.B. zu nennen:
➢ 3-Benzylidencampher bzw. 3-Benzylidennorcampher und dessen Derivate, z.B. 3-(4-Methylbenzyliden)campher wie in der **EP 0693471 B1** beschrieben;
➢ 4-Aminobenzoesäurederivate, vorzugsweise 4-(Dimethylamino)benzoesäure-2-ethylhexylester, 4-(Dimethylamino)benzoesäure-2-octylester und 4-(Dimethylamino)benzoesäureamylester;
➢ Ester der Zimtsäure, vorzugsweise 4-Methoxyzimtsäure-2-ethylhexylester, 4-Methoxyzimtsäurepropylester, 4-Methoxyzimtsäureisoamylester 2-Cyano-3,3-phenylzimtsäure-2-ethylhexylester (Octocrylene);
➢ Ester der Salicylsäure, vorzugsweise Salicylsäure-2-ethylhexylester, Salicylsäure-4-isopropylbenzylester, Salicylsäurehomomenthylester;
➢Derivate des Benzophenons, vorzugsweise 2-Hydroxy-4-methoxybenzophenon, 2-Hydroxy-4-methoxy-4'-methylbenzophenon, 2,2'-Dihydroxy-4-methoxybenzophenon;
➢Ester der Benzalmalonsäure, vorzugsweise 4-Methoxybenzmalonsäuredi-2-ethylhexylester;
➢ Triazinderivate, wie z.B. 2,4,6-Trianilino-(p-carbo-2'-ethyl-1'-hexyloxy)-1,3,5-triazin und Octyl Triazon, wie in der **EP 0818450 A1** beschrieben oder Dioctyl Butamido Triazone (Uvasorb® HEB);
➢ Propan-1,3-dione, wie z.B. 1-(4-tert.Butylphenyl)-3-(4'methoxyphenyl)propan-1,3-dion;
➢ Ketotricyclo(5.2.1.0)decan-Derivate, wie in der **EP 0694521 B1** beschrieben.

Als wasserlösliche Substanzen kommen in Frage:
➢ 2-Phenylbenzimidazol-5-sulfonsäure und deren Alkali-, Erdalkali-, Ammonium-, Alkylammonium-, Alkanolammonium- und Glucammoniumsalze;
➢ Sulfonsäurederivate von Benzophenonen, vorzugsweise 2-Hydroxy-4-methoxybenzophenon-5-sulfonsäure und ihre Salze;
➢Sulfonsäurederivate des 3-Benzylidencamphers, wie z.B. 4-(2-Oxo-3-bornylidenmethyl)benzolsulfonsäure und 2-Methyl-5-(2-oxo-3-bornyliden)sulfonsäure und deren Salze.

Als typische UV-A-Filter kommen insbesondere Derivate des Benzoylmethans in Frage, wie beispielsweise 1-(4'-tert.Butylphenyl)-3-(4'-methoxyphenyl)propan-1,3-dion, 4-tert.-Butyl-4'-methoxydibenzoylmethan (Parsol 1789), 1-Phenyl-3-(4'-isopropylphenyl)-propan-1,3-dion sowie Enaminverbindungen, wie beschrieben in der **DE 19712033 A1** (BASF). Die UV-A und UV-B-Filter können selbstverständlich auch in Mischungen eingesetzt werden. Neben den genannten löslichen Stoffen kommen für diesen Zweck auch unlösliche Lichtschutzpigmente, nämlich feindisperse Metalloxide bzw. Salze in Frage. Beispiele für geeignete Metalloxide sind insbesondere Zinkoxid und Titandioxid und daneben Oxide des Eisens, Zirkoniums, Siliciums, Mangans, Aluminiums und Cers sowie deren Gemische. Als Salze können Silicate (Talk), Bariumsulfat oder Zinkstearat eingesetzt werden. Die Oxide und Salze werden in Form der Pigmente für hautpflegende und hautschützende Emulsionen und dekorative Kosmetik verwendet. Die Partikel sollten dabei einen mittleren Durchmesser von weniger als 100 nm, vorzugsweise zwischen 5 und 50 nm und insbesondere zwischen 15 und 30 nm aufweisen. Sie können eine sphärische Form aufweisen, es können jedoch auch solche Partikel zum Einsatz kommen, die eine ellipsoide oder in sonstiger Weise von der sphärischen Gestalt abweichende Form besitzen. Die Pigmente können auch oberflächenbehandelt, d.h. hydrophilisiert oder hydrophobiert vorliegen. Typische Beispiele sind gecoatete Titandioxide, wie z.B. Titandioxid T 805 (Degussa) oder Eusolex® T2000 (Merck). Als hydrophobe Coatingmittel kommen dabei vor allem Silicone und dabei speziell Trialkoxyoctylsilane oder Simethicone in Frage. In Sonnenschutzmitteln werden bevorzugt sogenannte Mikro- oder Nanopigmente eingesetzt. Vorzugsweise wird mikronisiertes Zinkoxid verwendet. Weitere geeignete UV-Lichtschutzfilter sind der Übersicht von P.Finkel in **SÖFW-Journal 122, 543 (1996)** zu entnehmen.

Neben den beiden vorgenannten Gruppen primärer Lichtschutzstoffe können auch sekundäre Lichtschutzmittel vom Typ der **Antioxidantien** eingesetzt werden, die die photochemische Reaktionskette unterbrechen, welche ausgelöst wird, wenn UV-Strahlung in die Haut eindringt. Typische Beispiele hierfür sind Aminosäuren (z.B. Glycin, Histidin, Tyrosin, Tryptophan) und deren Derivate, Imidazole (z.B. Urocaninsäure) und deren Derivate, Peptide wie D,L-Carnosin, D-Carnosin, L-Carnosin und deren Derivate (z.B. Anserin), Carotinoide, Carotine (z.B. α-Carotin, β-Carotin, Lycopin) und deren Derivate, Chlorogensäure und deren Derivate, Liponsäure und deren Derivate (z.B. Dihydroliponsäure), Aurothioglucose, Propylthiouracil und andere Thiole (z.B. Thioredoxin, Glutathion, Cystein, Cystin, Cystamin und deren Glycosyl-, N-Acetyl-, Methyl-, Ethyl-, Propyl-, Amyl-, Butyl- und Lauryl-, Palmitoyl-, Oleyl-, γ-Linoleyl-, Cholesteryl- und Glycerylester) sowie deren Salze, Dilaurylthiodipropionat, Distearylthiodipropionat, Thiodipropionsäure und deren Derivate (Ester, Ether, Peptide, Lipide, Nukleotide, Nukleoside und Salze) sowie Sulfoximinverbindungen (z.B. Buthioninsulfoximine, Homocysteinsulfoximin, Butioninsulfone, Penta-, Hexa-, Heptathioninsulfoximin) in sehr geringen verträglichen Dosierungen (z.B. pmol bis µmol/kg), ferner (Metall)-Chelatoren (z.B. α-Hydroxyfettsäuren, Palmitinsäure, Phytinsäure, Lactoferrin), α-Hydroxysäuren (z.B. Citronensäure, Milchsäure, Äpfelsäure), Huminsäure, Gallensäure, Gallenextrakte, Bilirubin, Biliverdin, EDTA, EGTA und deren Derivate, ungesättigte Fettsäuren und deren Derivate (z.B. γ-Linolensäure, Linolsäure, Ölsäure), Folsäure und deren Derivate, Ubichinon und Ubichinol und deren Derivate, Vitamin C und Derivate (z.B. Ascorbylpalmitat, Mg-Ascorbylphosphat, Ascorbylacetat), Lipoinsäure, Tocopherole und Derivate (z.B. Vitamin-E-acetat), Vitamin A und Derivate (Vitamin-A-palmitat) sowie Koniferylbenzoat des Benzoeharzes, Rutinsäure und deren Derivate, α-Glycosylrutin, Ferulasäure, Furfurylidenglucitol, Carnosin, Butylhydroxytoluol, Butylhydroxyanisol, Nordihydroguajakharzsäure, Nordihydroguajaretsäure, Trihydroxybutyrophenon, Harnsäure und deren Derivate, Mannose und deren Derivate, Superoxid-Dismutase, Zink und dessen Derivate (z.B. ZnO, ZnSO₄) Selen und dessen Derivate (z.B. Selen-Methionin), Stilbene und deren Derivate (z.B. Stilbenoxid, trans-Stilbenoxid) und die erfindungsgemäß geeigneten Derivate (Salze, Ester, Ether, Zucker, Nukleotide, Nukleoside, Peptide und Lipide) dieser genannten Wirkstoffe.

Als **Konservierungsmittel** eignen sich beispielsweise Phenoxyethanol, Formaldehydlösung, Parabene, Pentandiol oder Sorbinsäure sowie die in Anlage 6, Teil A und B der Kosmetikverordnung aufgeführten weiteren Stoffklassen. Als **Insekten-Repellentien** kommen N,N-Diethyl-m-toluamid, 1,2-Pentandiol oder Ethyl Butylacetylaminopropionate in Frage, als **Selbstbräuner** eignet sich Dihydroxyaceton. Als **Tyrosinhinbitoren**, die die Bildung von Melanin verhindern und Anwendung in Depigmentierungsmitteln finden, kommen beispielsweise Arbutin, Kojisäure, Cumarinsäure und Ascorbinsäure (Vitamin C) in Frage.

Als **Parfümöle** seien genannt Gemische aus natürlichen und synthetischen Riechstoffen. Natürliche Riechstoffe sind Extrakte von Blüten (Lilie, Lavendel, Rosen, Jasmin, Neroli, Ylang-Ylang), Stengeln und Blättern (Geranium, Patchouli, Petitgrain), Früchten (Anis, Koriander, Kümmel, Wacholder), Fruchtschalen (Bergamotte, Zitrone, Orangen), Wurzeln (Macis, Angelica, Sellerie, Kardamon, Costus, Iris, Calmus), Hölzern (Pinien-, Sandel-, Guajak-, Zedern-, Rosenholz), Kräutern und Gräsern (Estragon, Lemongras, Salbei, Thymian), Nadeln und Zweigen (Fichte, Tanne, Kiefer, Latschen), Harzen und Balsamen (Galbanum, Elemi, Benzoe, Myrrhe, Olibanum, Opoponax). Weiterhin kommen tierische Rohstoffe in Frage, wie beispielsweise Zibet und Castoreum. Typische synthetische Riechstoffverbindungen sind Produkte vom Typ der Ester, Ether, Aldehyde, Ketone, Alkohole und Kohlenwasserstoffe. Riechstoffverbindungen vom Typ der Ester sind z.B. Benzylacetat, Phenoxyethylisobutyrat, p-tert.-Butylcyclohexylacetat, Linalylacetat, Dimethylbenzylcarbinylacetat, Phenylethylacetat, Linalylbenzoat, Benzylformiat, Ethylmethylphenylglycinat, Allylcyclohexylpropionat, Styrallylpropionat und Benzylsalicylat. Zu den Ethern zählen beispielsweise Benzylethylether, zu den Aldehyden z.B. die linearen Alkanale mit 8 bis 18 Kohlenstoffatomen, Citral, Citronellal, Citronellyloxyacetaldehyd, Cyclamenaldehyd, Hydroxycitronellal, Lilial und Bourgeonal, zu den Ketonen z.B. die Jonone, ∝-Isomethylionon und Methylcedrylketon, zu den Alkoholen Anethol, Citronellol, Eugenol, Isoeugenol, Geraniol, Linalool, Phenylethylalkohol und Terpineol, zu den Kohlenwasserstoffen gehören hauptsächlich die Terpene und Balsame. Bevorzugt werden jedoch Mischungen verschiedener Riechstoffe verwendet, die gemeinsam eine ansprechende Duftnote erzeugen. Auch ätherische Öle geringerer Flüchtigkeit, die meist als Aromakomponenten verwendet werden, eignen sich als Parfümöle, z.B. Salbeiöl, Kamillenöl, Nelkenöl, Melissenöl, Minzenöl, Zimtblätteröl, Lindenblütenöl, Wacholderbeerenöl, Vetiveröl, Olibanöl, Galbanumöl, Labolanumöl und Lavandinöl. Vorzugsweise werden Bergamotteöl, Dihydromyrcenol, Lilial, Lyral, Citronellol, Phenylethylalkohol, α-Hexylzimtaldehyd, Geraniol, Benzylaceton, Cyclamenaldehyd, Linalool, Boisambrene Forte, Ambroxan, Indol, Hedione, Sandelice, Citronenöl, Mandarinenöl, Orangenöl, Allylamylglycolat, Cyclovertal, Lavandinöl, Muskateller Salbeiöl, β-Damascone, Geraniumöl Bourbon, Cyclohexylsalicylat, Vertofix Coeur, Iso-E-Super, Fixolide NP, Evernyl, Iraldein gamma, Phenylessigsäure, Geranylacetat, Benzylacetat, Rosenoxid, Romilllat, Irotyl und Floramat allein oder in Mischungen, eingesetzt.

Als **Farbstoffe** können die für kosmetische Zwecke geeigneten und zugelassenen Substanzen verwendet werden, wie sie beispielsweise in der Publikation "**Kosmetische Färbemittel" der Farbstoffkommission der Deutschen Forschungsgemeinschaft, Verlag Chemie, Weinheim, 1984, S.81-106** zusammengestellt sind. Diese Wirkstoffe können auch ausschließlich aus ästhetischen Gründen in den Kapseln enthalten und nicht für eine kontrollierte Freigabe vorgesehen sein.

### Wirkstoffe für Detergensanwendungen

Bei Mikrokapselanwendungen im Bereich der Detergentien, insbesondere bei Wasch- und Reinigungsmitteln besteht ebenfalls der Wunsch, den Kontakt der verschiedenen Einsatzstoffe miteinander zu verhindern. So ist es sinnvoll, chemisch empfindliche Stoffe, wie beispielsweise Parfümöle oder optische Aufheller zu verkapseln, um deren Aktivität beispielsweise in Chlor- oder Peroxidbleichlaugen auch bei längerer Lagerung sicherzustellen. Man nutzt jedoch beispielsweise auch den Effekt, daß die Bleiche von Textilien in der Regel nicht zu Beginn des Waschprozesses, sondern erst in dessen Verlauf stattfindet und stellt mit der durch mechanische Einwirkung auf die Mikrokapseln verzögerten Freisetzung sicher, daß die Bleichmittel zum richtigen Zeitpunkt ihre volle Wirkung entfalten. Demzufolge kommen als Wirkstoffe, die es für Detergensanwendungen zu verkapseln gilt, vor allem Bleichmittel, Bleichaktivatoren, Enzyme, Vergrauungsinhibitoren, optische Aufheller sowie (chlor- bzw. peroxidstabile) Parfüm- und Farbstoffe in Frage.

Unter den als **Bleichmittel** dienenden, in Wasser Wasserstoffperoxid liefernden Verbindungen haben das Natriumperborat-Tetrahydrat und das Natriumperborat-Monohydrat eine besondere Bedeutung. Weitere Bleichmittel sind beispielsweise Peroxycarbonat, Citratperhydrate sowie Salze der Persäuren, wie Perbenzoate, Peroxyphthalate oder Diperoxydodecandisäure. Sie werden üblicherweise in Mengen von 8 bis 25 Gew.-% eingesetzt. Bevorzugt ist der Einsatz von Natriumperborat-Monohydrat in Mengen von 10 bis 20 Gew.-% und insbesondere von 10 bis 15 Gew.-%. Durch seine Fähigkeit, unter Ausbildung des Tetrahydrats freies Wasser binden zu können, trägt es zur Erhöhung der Stabilität des Mittels bei.

Beispiele für geeignete **Bleichaktivoren** sind mit Wasserstoffperoxid organische Persäuren bildende N-Acyl- bzw. O-Acyl-Verbindungen, vorzugsweise N,N'-tetraacylierte Diamine, ferner Carbonsäureanhydride und Ester von Polyolen wie Glucosepentaacetat. Der Gehalt der bleichmittelhaltigen Mittel an Bleichaktivatoren liegt in dem üblichen Bereich, vorzugsweise zwischen 1 und 10 Gew.-% und insbesondere zwischen 3 und 8 Gew.-%. Besonders bevorzugte Bleichaktivatoren sind N,N,N',N'-Tetraacetylethylendiamin und 1,5-Diacetyl-2,4-dioxo-hexahydro-1,3,5-triazin.

Als **Enzyme** kommen solche aus der Klasse der Proteasen, Lipasen, Amylasen, Cellulasen bzw. deren Gemische in Frage. Besonders gut geeignet sind aus Bakterienstämmen oder Pilzen, wie Bacillus subtilis, Bacillus licheniformis und Streptomyces griseus gewonnene enzymatische Wirkstoffe. Vorzugsweise werden Proteasen vom Subtilisin-Typ und insbesondere Proteasen, die aus Bacillus lentus gewonnen werden, eingesetzt. Ihr Anteil kann etwa 0,2 bis etwa 2 Gew.-% betragen. Die Enzyme können an Trägerstoffen adsorbiert und/oder in Hüllsubstanzen eingebettet sein, um sie gegen vorzeitige Zersetzung zu schützen. Zusätzlich zu den mono- und polyfunktionellen Alkoholen und den Phosphonaten können die Mittel weitere Enzymstabilisatoren enthalten. Beispielsweise können 0,5 bis 1 Gew.-% Natriumformiat eingesetzt werden. Möglich ist auch der Einsatz von Proteasen, die mit löslichen Calciumsalzen und einem Calciumgehalt von vorzugsweise etwa 1,2-Gew.-%, bezogen auf das Enzym, stabilisiert sind. Besonders vorteilhaft ist jedoch der Einsatz von Borverbindungen, beispielsweise von Borsäure, Boroxid, Borax und anderen Alkalimetallboraten wie den Salzen der Orthoborsäure (H₃BO₃), der Metaborsäure (HBO₂) und der Pyroborsäure (Tetraborsäure H₂B4O₇).

Geeignete **Vergrauungsinhibitoren** sind wasserlösliche Kolloide meist organischer Natur, beispielsweise die wasserlöslichen Salze polymerer Carbonsäuren, Leim, Gelatine, Salze von Ethercarbonsäuren oder Ethersulfonsäuren der Stärke oder der Cellulose oder Salze von sauren Schwefelsäureestem der Cellulose oder der Stärke. Auch wasserlösliche, saure Gruppen enthaltende Polyamide sind für diesen Zweck geeignet. Weiterhin lassen sich lösliche Stärkepräparate und andere als die obengenannten Stärkeprodukte verwenden, z.B. abgebaute Stärke, Aldehydstärken usw.. Auch Polyvinylpyrrolidon ist brauchbar. Bevorzugt werden jedoch Celluloseether, wie Carboxymethylcellulose, Methylcellulose, Hydroxyalkylcellulose und Mischether, wie Methylhydroxyethylcellulose, Methylhydroxypropylcellulose, Methylcarboxymethylcellulose und deren Gemische sowie Polyvinylpyrrolidon, beispielsweise in Mengen von 0,1 bis 99 und vorzugsweise 1 bis 5 Gew.-%, bezogen auf die Mittel.

Als **optische Aufheller** können Derivate der Diaminostilbendisulfonsäure bzw. deren Alkalimetallsalze eingesetzt werden. Geeignet sind z.B. Salze der 4,4'-Bis(2-anilino-4-morpholino-1,3,5-triazinyl-6-amino)stilben-2,2'-disulfonsäure oder gleichartig aufgebaute Verbindungen, die anstelle der Morpholino-Gruppe eine Diethanolaminogruppe, eine Methylaminogruppe, eine Anilinogruppe oder eine 2-Methoxyethylaminogruppe tragen. Weiterhin können Aufheller vom Typ der substituierten Diphenylstyryle anwesend sein, z.B. die Alkalisalze des 4,4'-Bis(2-sulfostyryl)-diphenyls, 4,4'-Bis(4-chlor-3-sulfostyryl)-diphenyls, oder 4-(4-Chlorstyryl)-4'-(2-sulfostyryl)-diphenyls. Auch Gemische der vorgenannten Aufheller können verwendet werden. Ein besonders bevorzugter Farbstoff ist Tinolux® (Handelsprodukt der Ciba-Geigy).

Beispiele für **aktivchlorbeständige Duftstoffe** sind Citronellol (3,7-Dimethyl-6-octen-1-ol), Dimethyloctanol (3,7-Dimethyloctanol-1), Hydroxycitronellol (3,7-Dimethyloctane-1,7-diol), Mugol (3,7-Dimethyl-4,6-octatrien-3-ol), Mirsenol (2-Methyl-6-methylen-7-octen-2-ol), Terpinolen (p-Mentho-1,4(8)-dien), Ethyl-2-methylbutyrat, Phenylpropylalkohol, Galaxolid (1,3,4,6,7,8-hexahydro-4,6,6,7,8,8,-hexamethylcyclopental-2-benzopyran, Tonalid (7-Acetyl-1,1,3,4,4,6-hexamethyltetrahydronaphthalin), Rosenoxid, Linaloloxid, 2,6-Dimethyl-3-octanol, Tetrahydroethyllinalool, Tetrahydroethyllinalylacetat, o-sec-Butylcyclohexylacetat und Isolonediphorenepoxid sowie Isoborneal, Dihydroterpenöl, Isobornylacetat, Dihydroterpenylacetat). Weitere geeignete Duftstoffe sind die in der Europäischen Patentanmeldung **EP 0622451 A1** (Procter & Gamble) in den Spalten 3 und 4 genannten Stoffe.

Als **Farbpigmente** kommen neben anorganischen Stoffen, wie beispielsweise Eisen- oder Wismutoxiden, vor allem grüne Chlorophthalocyanine (Pigmosol® Grün, Hostaphine® Grün), gelbes Solar Yellow BG 300 (Sandoz), blaues Chlorophthalocyanin (Hostaphine® Blau) oder Cosmenyl® Blau in Frage.

### Chitosane

Chitosane stellen Biopolymere dar und werden zur Gruppe der Hydrokolloide gezählt. Chemisch betrachtet handelt es sich um partiell deacetylierte Chitine unterschiedlichen Molekulargewichtes, die den folgenden - idealisierten - Monomerbaustein enthalten:

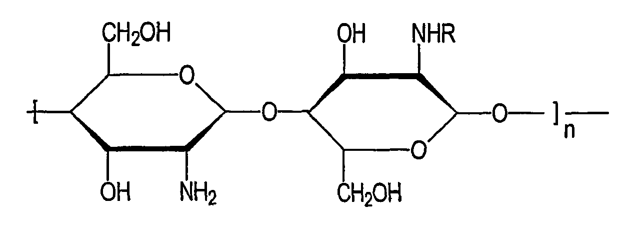

Im Gegensatz zu den meisten Hydrokolloiden, die im Bereich biologischer pH-Werte negativ geladen sind, stellen Chitosane unter diesen Bedingungen kationische Biopolymere dar. Die positiv geladenen Chitosane können mit entgegengesetzt geladenen Oberflächen in Wechselwirkung treten und werden daher in kosmetischen Haar- und Körperpflegemitteln sowie pharmazeutischen Zubereitungen eingesetzt (vgl. **Ullmann's Encyclopedia of Industrial Chemistry, 5th Ed., Vol. A6, Weinheim, Verlag Chemie, 1986, S. 231-232).** Übersichten zu diesem Thema sind auch beispielsweise von B. Gesslein et al. in **HAPPI 27, 57 (1990),** O. Skaugrud in **Drug Cosm.Ind. 148, 24 (1991)** und E. Onsoyen et al, in **Seifen-Öle-Fette-Wachse 117, 633 (1991)** erschienen. Zur Herstellung der Chitosane geht man von Chitin, vorzugsweise den Schalenresten von Krustentieren aus, die als billige Rohstoffe in großen Mengen zur Verfügung stehen. Das Chitin wird dabei in einem Verfahren, das erstmals von Hackmann et al. beschrieben worden ist, üblicherweise zunächst durch Zusatz von Basen deproteiniert, durch Zugabe von Mineralsäuren demineralisiert und schließlich durch Zugabe von starken Basen deacetyliert, wobei die Molekulargewichte über ein breites Spektrum verteilt sein können. Entsprechende Verfahren sind beispielsweise aus **Makromol. Chem. 177, 3589 (1976)** oder der französischen Patentanmeldung **FR 2701266 A** bekannt. Vorzugsweise werden solche Typen eingesetzt, wie sie in den deutschen Patentanmeldungen **DE 4442987 A1** und **DE 19537001 A1** (Henkel) offenbart werden und die ein durchschnittliches Molekulargewicht von 10.000 bis 500.000 bzw. 800.000 bis 1.200.000 Dalton aufweisen und/oder eine Viskosität nach Brookfield (1 Gew.-%ig in Glycolsäure) unterhalb von 5000 mPas, einen Deacetylierungsgrad im Bereich von 80 bis 88 % und einem Aschegehalt von weniger als 0,3 Gew.-% besitzen. Aus Gründen der besseren Wasserlöslichkeit werden die Chitosane in der Regel in Form ihrer Salze, vorzugsweise als Glycolate eingesetzt.

### Herstellung der Mikrokapseln

Alle Verfahren zur Herstellung von Mikro- und/oder Nanokapseln, bei dem die gebildeten Kapseln als Dispersion mit einem Ölkörper in der äußeren Phase vorliegen, die dann mit dem ölabsorbierenden Hilfsstoff vermischt werden kann, sind grundsätzlich anwendbar. Dabei wird die W/O-Emulsionsmethode mit verbundener Gelierung durch Temperaturabsenkung besonders hervorgehoben.

Zur Herstellung der erfindungsgemäßen hydrophobierten Pulver soll zunächst durch inniges Vermischen von Wirkstoffen und Hilfsstoffen eine wäßrige Lösung oder Dispersion hergestellt werden. Die hydrophilen Polymere, die zur Bildung der Matrix dienen, werden durch Erwärmen über ihren Gelpunkt darin gelöst oder bei Bedarf getrennt unter Erhitzen im wäßrigen Lösungsmittel gelöst und mit der erwärmten Lösung der anderen Wirk- und Hilfsstoffe zusammengeführt. Falls erforderlich, können der wäßrigen Lösung neben Wasser auch andere hydrophile Lösungsmittel wie z. B. Ethanol, Isopropanol zugefügt werden. Diese wäßrige Lösung oder Dispersion wird dann in einem Öl, das bis über die Geltemperatur des matrixbildenden Polymers erwärmt wurde, unter intensivem Rühren dispergiert. Bis zu diesem Schritt finden alle Vorgänge oberhalb der Geltemperatur des hydrophilen Polymers statt. Nach der homogenen Emulsionsbildung wird der Ansatz auf eine Temperatur unterhalb des Gelpunktes des hydrophilen Polymers abgekühlt, und es entsteht eine ölige Dispersion mit einem Feststoffanteil aus Mikro- und/oder Nanokapseln und einer äußeren Phase des Öles. Zur Isolierung der Mikro-und/oder Nanokapseln wird überschüssiges Öl langsam abdekantiert, so dass der Ölanteil der nun erhaltenen Dispersion ungefähr 20 bis 2 Gew. %, vorzugsweise 10 bis 5 Gew. % beträgt.

Die anhaftende Ölschicht wird jetzt adsorbiert durch intensives Mischen der Dispersion mit einem ölabsorbierenden, pulverigen Hilfsstoff. Das Mischen erfolgt mit handelsüblichen Pulvermischern bis eine homogene Pulvermischung erreicht ist.

Im Mittel zeigen die im Pulver enthaltenen Mikro- und/oder Nanokapseln einen Durchmesser im Bereich von 0,1 bis 500, vorzugsweise 50 bis 100 µm, besonders bevorzugt 10 bis 50 µm. Der Partikeldurchmesser wird bestimmt durch Laserdiffraktometrie (Malvern Instruments), die Messungen ergeben eine Volumenverteilung.

Die Mikro- und/oder Nanokapseln können bei einer Verkapselung von Wirkstoffen eine Wirkstoffbeladung von 0,1 bis 50, vorzugsweise 1 bis 25 und insbesondere 5 bis 10 Gew.-% aufweisen.

### Gewerbliche Anwendbarkeit

Mikro- und/oder Nanokapseln in Form des hydrophobierten Pulvers können zu vielfältigen Zwecken eingesetzt werden. Vorwiegend dienen Sie der kontrollierten Freisetzung und dem Schutz der eingekapselten bzw. eingelagerten Stoffe. Diese Schutzfunktion schließt die Oxidation durch Luftsauerstoff ein, den Schutz hygroskopischen Materials, Schutz vor UV-Strahleneinwirkung oder auch die Separation nicht miteinander kompatibler Inhaltsstoffe ein, die auf diese Weise getrennt gelagert werden können. Somit steigt insbesondere die Stabilität der verarbeiteten Inhaltsstoffe. Des weiteren kann eine Maskierung der Inhaltsstoffe beabsichtigt sein, bei der Geruch, Geschmack und/oder Aussehen überdeckt werden sollen.
Die Auswahl der Wirkstoffe, die in den neuen Mikrokapseln eingeschlossen sind, ist an sich unkritisch. Vorzugsweise handelt es sich um Stoffe, die erst durch mechanische Zerstörung der Mikrokapseln freigesetzt werden. In diesen Fällen kommt den Mikrokapseln die Aufgabe zu, den Kontakt zwischen äußerer Umgebung und Wirkstoff und damit eine chemische Reaktion bzw. einen Abbau zu verhindern. Es kann es sein, daß die in der Kapsel eingeschlossenen Stoffe überhaupt nicht freigesetzt werden sollen und ausschließlich dem Zweck dienen, der Zubereitung ein ästhetisches Äußeres zu verleihen; dies trifft beispielsweise vielfach für Farbstoffe zu. Es ist natürlich klar, daß diese Einsatzformen auch nebeneinander bestehen können. Insbesondere ist es möglich, beispielsweise einen Duftstoff für die spätere Freisetzung zusammen mit einem Farbpigment zu verkapseln, welches der Kapsel ein besonderes Aussehen verleiht.

Zur Verarbeitung in kosmetischen und/oder pharmazeutischen Mitteln ist es häufig vorteilhaft, wenn die Mikro- und/oder Nanokapseln in Pulverform vorliegen.
Die erfindungsgemäßen Pulverformulierungen können mit den unterschiedlichsten Wirkstoffen beladen werden, welche sie zeitverzögert und unter mechanischem Druck freisetzen. Gegenüber bekannten Formulierungen zeichnen sie sich durch eine höhere Stabilität besonders bei weiterer mechanischer Verarbeitung aus. Es ist daher auch möglich, die hydrophobierten Pulver in Wasch-, Spül-, Reinigungs- und Avivagemitteln, aber auch zur Herstellung von Lebensmitteln einzusetzen. Üblicherweise können die Pulver in Mengen von 0,01 bis 100, vorzugsweise 0,1 bis 50 und insbesondere 1 bis 25 Gew.-% - bezogen auf die Zubereitungen - eingesetzt werden. Vorzugsweise dienen sie zur Herstellung von kosmetischen Produkten, wie z.B. Haarshampoos, Haarlotionen, Schaumbäder, Duschbäder, Cremes, Gele, Lotionen, alkoholische und wäßrig/alkoholische Lösungen, Emulsionen, Wachs/Fett-Massen, Stiftpräparaten, Pudern und insbesondere dekorativen kosmetischen Zubereitungen, wie z.B. Make-Ups, Rouges, Lippenstiften, Kajals, Lidschatten, Mascaras und Nagellacken. Besonders in wasserfreie Mittel, Zubereitungen, die weniger als 5 Gew.%, vorzugsweise weniger als 3 Gew. % und besonders bevorzugt weniger als 1 Gew. % Wasser enthalten, lassen sich die hydrophobierten Pulver hervorragend einarbeiten.
Diese Mittel können ferner als weitere Hilfs- und Zusatzstoffe milde Tenside, Ölkörper, Emulgatoren, Überfettungsmittel, Perlglanzwachse, Konsistenzgeber, Verdickungsmittel, Polymere, Siliconverbindungen, Fette, Wachse, Lecithine, Phospholipide, Stabilisatoren, biogene Wirkstoffe, Deodorantien, Antitranspirantien, Antischuppenmittel, Filmbildner, Quellmittel, UV-Lichtschutzfaktoren, Antioxidantien, Hydrotrope, Konservierungsmittel, Insektenrepellentien, Selbstbräuner, Tyrosininhibitoren (Depigmentierungsmittel), Solubilisatoren, Parfümöle, Farbstoffe und dergleichen enthalten. Die Mehrzahl dieser Stoffe stellen auch potentielle Wirkstoffe dar, mit denen die Mikrokapseln beladen werden können und wurden in diesem Kapitel bereits näher erläutert.

### Beispiele

### Beispiel 1.

### 1a: Herstellung der hydrophilen Partikel

| | **Phase A** | | **Gew. %** |
|---|---|---|---|
| 1 | Agar | * | 1,50 |
| 2 | Konservierungsmittel | | q.s. |
| 3 | Titandioxid | | 3,00 |
| 4 | Photonyl LS® | ** | 30,00 |
| 5 | Wasser | | Ad 100,0 |
| | | | |

| | **Phase B** | | |
|---|---|---|---|
| 6 | Cetiol® OE | Dicaprylyl Ether | 99,50 |
| 7 | Dehymuls® PGPH | Polyglyceryl-2-Dipolyhydroxystearate | 0,50 |

| | | | |
|---|---|---|---|
| * Granulated Agar-Agar, produced by Merck, Darmstadt, Artikelnummer: 1.01614 | | | |
| ** Handelsprodukt der Firma Laboratoires Sérobiologiques bestehend aus: Arginin, Natriumadenosintriphosphat, Mannitol, Pyridoxin-HCl, RNA, Histidin-HCl, Phenylalanin und Tyrosin. | | | |

Agar wird im konservierten Wasser bei 90°C gelöst und nachfolgend auf 75°C abgekühlt, Titandioxid und Photonyl® LS werden dann unter Rühren zugefügt. Die Lösung wird auf 55°C gehalten.
Cetiol® OE und Dehymuls® PGHG werden gemischt und auf 40 - 42°C erhitzt. Ein Teil der Phase A wird in 3,3 Teilen der Phase B unter Rühren mit dem Ultra-Turrax bei 40 - 42°C über 5 Minuten dispergiert.
Danach wird die Dispersion unter Rühren auf Raumtemperatur (25°C) abgekühlt, so dass die Tropfen der Phase A zu Mikrokapseln erhärten. Durch Dekantieren werden die Mikrokapseln isoliert. Jedoch besteht die erhaltene Formulierung aufgrund der anhaftenden Ölphase aus einer öligen Dispersion mit hohem Feststoffanteil (Phase A: Gew. 93%, Phase B: 7 Gew. %).

### 1b: Herstellung des hydrophobierten Pulvers

| | **Bestandteile** | | **Gew. %** |
|---|---|---|---|
| 8 | Mikrokapseldispersion | aus 1a | 75 |
| 9 | Polytrap® 6603* | Acrylat-Copolymer | 25 |

| | | | |
|---|---|---|---|
| *Hersteller: Advanced Polymer systems, Vertrieb: Dow Corning | | | |

Die ölige Mikrokapseldispersion wird dann mit Polytrap® 6603 gemischt bis ein homogenes Pulver entstanden ist. Der Gehalt an Photonyl® LS im hydrophobierten Pulver beträgt 20 Gew. %.

## Patentansprüche

1. Hydrophobiertes Pulver bestehend aus Mikro- und/oder Nanokapseln **dadurch** erhältlich, dass man
(a) eine wäßrige Lösung mindestens eines Polymers in Gegenwart eines W/O-Emulgators in einem Öl bei einer Temperatur oberhalb des Gelpunktes der Polymerlösung dispergiert,
(b) die Dispersion unter Rühren auf eine Temperatur unterhalb des Gelpunktes abkühlt,
(c) die dabei entstandenen Mikro- und/oder Nanokapseln durch Dekantieren isoliert, und
(d) die so erhaltene ölige Dispersion mit einem ölabsorbierenden Hilfsstoff versetzt.

2. Verfahren zur Herstellung von hydrophobierten Pulvern bestehend aus Mikro- und/oder Nanokapseln , bei dem man
(a) eine erwärmte wäßrige Lösung mindestens eines Polymers in Gegenwart eines W/O-Emulgators in einem Öl bei einer Temperatur oberhalb des Gelpunktes der Polymerlösung dispergiert,
(b) die Dispersion unter Rühren auf eine Temperatur unterhalb des Gelpunktes abkühlt,
(c) die dabei entstandenen Mikro- und/oder Nanokapseln durch Dekantieren isoliert, und
(d) die so erhaltene ölige Dispersion mit einem ölabsorbierenden Hilfsstoff versetzt.

3. Verfahren nach Anspruch 2, **dadurch gekennzeichnet, dass** man wäßrige Lösungen des Polymers einschließt, welche weitere Wirk- und/oder Hilfsstoffe enthalten, die bei der Kapselgewinnung in die Polymerhülle eingeschlossen werden.

4. Verfahren nach Anspruch 2 und/oder 3, **dadurch gekennzeichnet, dass** man wäßrige zu dispergierende Lösungen a) einsetzt, die Polymere enthalten ausgewählt aus der Gruppe, die gebildet wird von Gummi Arabicum, Agar-Agar, Agarose, Carrageen, Maltodextrine, Alginsäure bzw. ihre Salze, z.B. Natrium- oder Calciumalginat, Fette und Fettsäuren, Cetylalkohol, Collagen, Chitosan, Lecithine, Gelatine, Gluten, Albumin, Schellack, Polysaccaride, wie Stärke oder Dextran, Polypeptide, Proteinhydrolysate, Xanthan, Gellan Gum und Sucrose, chemisch modifizierte Cellulosen, insbesondere Celluloseester und -ether, z.B. Celluloseacetat, Ethylcellulose, Hydroxypropylcellulose, Hydroxypropylmethylcellulose und -phthalat, Methylcellulose und Carboxymethylcellulose, sowie Stärkederivate, insbesondere Stärkeether und -ester und Stearate, Polyacrylate, Polyamide, Polyvinylalkohol, Polyglycolate, Polyoxyethylene, Polylactate, Polyglutamate, Polyimide und Polyvinylpyrrolidon.

5. Verfahren nach mindestens einem der Ansprüche 2 bis 4, **dadurch gekennzeichnet, dass** die Mikro- und/oder Nanokapseln, die abdekantiert werden c) einen mittleren Teilchendurchmesser im Bereich von 0,1 bis 500 µm aufweisen.

6. Verfahren nach mindestens einem der Ansprüche 2 bis 5, **dadurch gekennzeichnet, dass** man ölabsorbierende Hilfsstoffe d) einsetzt, ausgewählt aus der Gruppe, die gebildet wird von DimethiconNinyldimethicone Crosspolymer, Stärke, Acrylat-Copolymeren, Methacrylatverbindungen, Polymethylmethacrylaten, Silicaten, Siliciumdioxid, Magnesiumstearat, Zinkstearat und Magnesiumcarbonat.

7. Verfahren nach mindestens einem der Ansprüche 2 bis 6, **dadurch gekennzeichnet, dass** man den ölabsorbierenden Hilfsstoff d) in Mengen von 5 bis 35 Gew. % bezogen auf die Menge des hydrophobierten Pulvers eingesetzt.

8. Verwendung von hydrophobierten Pulvern nach Anspruch 1 in kosmetischen und/oder pharmazeutischen Zubereitungen.

9. Verwendung von hydrophobierten Pulvern nach Anspruch 1 in Wasch- und Reinigungsmitteln.

10. Verwendung nach Anspruch 8 und 9, **dadurch gekennzeichnet, dass** man das hydrophobierte Pulver in Mengen von 0,01 bis 100 Gew.-% - bezogen auf die Zubereitungen - einsetzt.

## Claims

1. A hydrophobicized powder consisting of micro- and/or nanocapsules obtainable by
(a) dispersing an aqueous solution of at least one polymer in an oil in the presence of a w/o emulsifier at a temperature above the gel point of the polymer solution,
(b) cooling the dispersion while stirring to a temperature below the gel point,
(c) isolating the micro- and/or nanocapsules formed by decantation and
(d) adding an oil-absorbing auxiliary to the oily dispersion obtained.

2. A process for the production of hydrophobicized powders consisting of micro- and/or nanocapsules in which
(a) a heated aqueous solution of at least one polymer is dispersed in an oil in the presence of a w/o emulsifier at a temperature above the gel point of the polymer solution,
(b) the dispersion is cooled while stirring to a temperature below the gel point,
(c) the micro- and/or nanocapsules formed are isolated by decantation and
(d) an oil-absorbing auxiliary is added to the oily dispersion obtained.

3. A process as claimed in claim 2, **characterized in that** aqueous solutions of the polymer containing other active substances and/or auxiliaries encapsulated in the polymer membrane during production of the capsules are included.

4. A process as claimed in claim 2 and/or 3, **characterized in that** aqueous solutions a) to be dispersed which contain polymers selected from the group consisting of gum arabic, agar agar, agarose, carrageen, maltodextrins, alginic acid and salts thereof, for example sodium or calcium alginate, fats and fatty acids, cetyl alcohol, collagen, chitosan, lecithins, gelatin, gluten, albumin, shellac, polysaccharides, such as starch or dextran, polypeptides, protein hydrolyzates, xanthan, gellan gum and sucrose, chemically modified celluloses, more particularly cellulose esters and ethers, for example cellulose acetate, ethyl cellulose, hydroxypropyl cellulose, hydroxypropyl methyl cellulose and phthalate, methyl cellulose and carboxymethyl cellulose, and starch derivatives, more particularly starch ethers and esters, and stearates, polyacrylates, polyamides, polyvinyl alcohol, polyglycolates, polyoxyethylenes, polylactates, polyglutamates, polyimides and polyvinyl pyrrolidone are used.

5. A process as claimed in at least one of claims 2 to 4, **characterized in that** the micro- and/or nanocapsules decanted off c) have a mean particle diameter of 0.1 to 500 µm.

6. A process as claimed in at least one of claims 2 to 5, **characterized in that** oil-absorbing auxiliaries d) selected from the group consisting of dimethicone/vinyl dimethicone cross polymer, starch, acrylate copolymers, methacrylate compounds, polymethyl methacrylates, silicates, silicon dioxide, magnesium stearate, zinc stearate and magnesium carbonate are used.

7. A proess as claimed in at least one of claims 2 to 6, **characterized in that** the oil-absorbing auxiliary d) is used in quantities of 5 to 35% by weight, based on the quantity of hydrophobicized powder.

8. The use of the hydrophobicized powder claimed in claim 1 in cosmetic and/or pharmaceutical preparations.

9. The use of the hydrophobicized powder claimed in claim 1 in detergents.

10. The use claimed in claims 8 and 9, **characterized in that** the hydrophobicized powder is used in quantities of 0.01 to 100% by weight, based on the preparations.

## Revendications

1. Poudre rendue hydrophobe composée de micro- et/ou de nanocapsules, obtenue en ce que
(a) on disperse une solution aqueuse d'au moins un polymère en présence d'un émulsifiant eau-dans-huile dans une huile à une température supérieure au point de gélification de la solution polymère,
(b) on refroidit la dispersion sous agitation jusqu'à une température inférieure au point de gélification,
(c) les micro- et/ou nanocapsules ainsi formées sont isolées par décantation, et
(d) la dispersion huileuse ainsi obtenue est mélangée avec un agent auxiliaire absorbant l'huile.

2. Procédé de fabrication de poudres rendues hydrophobes composées de micro- et/ou de nanocapsules, dans lequel
(a) on disperse une solution aqueuse chauffée d'au moins un polymère en présence d'un émulsifiant eau-dans-huile dans une huile à une température supérieure au point de gélification de la solution polymère,
(b) on refroidit la dispersion sous agitation à une température inférieure au point de gélification,
(c) les micro- et/ou nanocapsules ainsi formées sont isolées par décantation, et
(d) la dispersion huileuse ainsi obtenue est mélangée avec un agent auxiliaire absorbant l'huile.

3. Procédé selon la revendication 2, **caractérisé en ce que** l'on inclut des solutions aqueuses du polymère qui contiennent d'autres agents actifs et auxiliaires qui sont incorporés dans l'enveloppe polymère lors de la réalisation des capsules.

4. Procédé selon la revendication 2 ou 3, **caractérisé en ce que** l'on utilise des solutions aqueuses (a) à disperser qui contiennent des polymères sélectionnés parmi le groupe formé de la gomme arabique, de l'agar agar, de l'agarose, de la carragénine, de la maltodextrine, de l'acide alginique ou de ses sels, par exemple l'alginate de sodium ou de calcium, des graisses et des acides gras, de l'alcool de cétyle, du collagène, du chitosan, de la lécithine, de la gélatine, du glutène, de l'albumine, de la gomme laque, des polysaccharides, comme l'amidon ou le dextrane, des polypeptides, des hydrolysats de protéine, du xanthane, de la gomme Kelco et du saccharose, des celluloses chimiquement modifiées, en particulier des esters et des éthers de cellulose, par exemple l'acétate de cellulose, l'éthylcellulose, l'hydroxypropylcellulose, l'hydroxypropylméthyl-cellulose et -phtalate, la méthylcellulose et la carboxyméthylcellulose, ainsi que des dérivés d'amidon, en particulier des éthers et des esters d'amidon, et des stéarates, du polyacrylate, du polyamide, de l'alcool de polyvinyle, des polyglycolates, des polyoxyéthylènes, des polylactates, des polyglutamates, des polyimides et de la polyvinylpyrrolidone.

5. Procédé selon au moins l'une des revendications 2 à 4, **caractérisé en ce que** les micro- et/ou nanocapsules qui sont décantées c) présentent un diamètre moyen de particules compris dans la gamme allant de 0,1 à 500 µm.

6. Procédé selon au moins l'une des revendications 2 à 5, **caractérisé en ce que** l'on utilise des agents auxiliaires absorbant l'huile (d) sélectionnés parmi le groupe formé des polymères réticulés de diméthicone/vinyldiméthicone, des amidons, des copolymères d'acrylate, des composés de méthacrylate, des méthacrylates de polyméthyle, des silicates, du dioxyde de silicium, du stéarate de magnésium, du stéarate de zinc et du carbonate de magnésium.

7. Procédé selon au moins l'une des revendications 2 à 6, **caractérisé en ce que** l'on utilise l'agent auxiliaire absorbant l'huile d) dans des quantités comprises entre 5 et 35 % en masse, sur la base de la quantité de la poudre rendue hydrophobe.

8. Utilisation de poudres rendues hydrophobes selon la revendication 1 dans des préparations cosmétiques et/ou pharmaceutiques.

9. Utilisation de poudres rendues hydrophobes selon la revendication 1 dans des produits lavants et nettoyants.

10. Utilisation selon la revendication 8 et 9, **caractérisée en ce que** l'on utilise la poudre rendue hydrophobe dans des quantités comprises entre 0,01 et 100 % en masse - sur la base des préparations finales.
